# FASCICULE DE BREVET EUROPEEN

(11) **EP 0 625 337 B1**
(45) Date de publication et mention de la délivrance du brevet: **16.07.1997**
(21) Numéro de dépôt: 94420141.7
(22) Date de dépôt: 16.05.1994
(51) Int. Cl.: A61B 17/60, A61B 17/58

(54) **Fixateur de rachis pour le maintien d'une colonne vertébrale**
Wirbelfixationsvorrichtung zum Halten der Wirbelsäule
Spinal fixator for holding the vertebral column

(30) Priorité: 17.05.1993 FR 9306175
(43) Date de publication de la demande: 23.11.1994
(73) Titulaire: TORNIER S.A., F-38330 Saint-Ismier (FR)
(72) Inventeur: Judet, Thierry, F-92410 Ville d'Avray (FR)
(74) Mandataire: Monnier, Guy

(56) Documents cités:
- EP-A- 0 007 393
- EP-A- 0 506 420
- WO-A-91/16020
- WO-A-91/19469
- DE-U- 9 302 700

## Description

La présente invention a trait à un fixateur de rachis pour l'alignement et le maintien d'une colonne vertébrale.

On connaît des fixateurs de ce genre qui comprennent généralement deux montages parallèles, de sorte que chacun comporte :
- une tige cylindrique dont la longueur est déterminée suivant la hauteur et le nombre des étages à réunir ;
- et plusieurs ensembles composés d'une bague, d'un écrou et d'une vis pédiculaire ou de crochets permettant la fixation des tiges sur les vertèbres.

D'autres fixateurs comprennent des plaques perforées de trous ou de lumières permettant le passage des vis s'implantant dans les pédicules. De tels fixateurs comportent certains inconvénients en ce qui concerne les risques de micro-mouvements ou de fragilité de la solidarisation qui subsistent soit entre la vis et la tige, soit entre les vis et la plaque.

De plus, les fixateurs par plaques imposent un entraxe pédiculaire pouvant ne pas s'adapter aux variations anatomiques.

On connaît d'après le brevet WO 91/16020 un fixateur de rachis comprenant une tige pleine reliant des vis pédiculaires entre elles par l'intermédiaire de bagues. L'extrémité supérieure de chaque bague est déformée par un capuchon fileté permettant le serrage de la tige à l'intérieur de la bague correspondante.

C'est à ces inconvénients qu'entend remédier plus spécialement la présente invention.

Le fixateur de rachis suivant la présente invention est constitué de deux ensembles sensiblement parallèles qui sont constitués chacun :
- d'une coulisse qui présente une forme en U dont le fond est percé d'une lumière débouchante ;
- de plusieurs bagues qui sont chacune percées d'une ouverture débouchante horizontale présentant un profil interne correspondant à celui de la coulisse, d'un alésage taraudé coaxial à un trou lisse ménagé dans le fond de la bague perpendiculairement à l'ouverture ;
- de vis pédiculaires à filet auto-taraudant et à tête sphérique permettant la mise en place et le positionnement de la coulisse dans la bague ;
- et de capuchons filetés vissés dans l'alésage taraudé de chaque bague et qui prennent appui chacun sur la partie supérieure de la tête de la vis de manière à assembler par serrage la bague et la coulisse, par l'intermédiaire de la tête de la vis.

Un mode d'exécution de la présente invention consiste en ce que la bague est pourvue d'un trou débouchant qui est prévu au-dessus de l'ouverture, mais perpendiculairement à celle-ci.

Un autre mode d'exécution preferé de la présente invention consiste en ce qu'il comprend une bague pourvue d'une ouverture horizontale débouchante en forme de U, un trou percé dans le fond de ladite bague, un alésage taraudé coaxial audit trou et une plaque horizontale qui est prévue au niveau du fond de ladite bague et dans le prolongement de l'ouverture.

Le dessin annexé, donné à titre d'exemple, permettra de mieux comprendre l'invention, les caractéristiques qu'elle présente et les avantages qu'elle est susceptible de procurer :
Fig. 1 est une vue en élévation illustrant les différents éléments qui composent le fixateur de rachis suivant l'invention.
Fig. 2 est une vue en perspective montrant en détail les différents éléments composant chaque ensemble du fixateur de rachis.
Fig. 3 est une coupe suivant III-III (fig. 1) représentant l'immobilisation d'un des ensembles du fixateur.
Fig. 4 est une coupe suivant IV-IV (fig. 1) montrant le dispositif de stabilisation transversale.
Fig. 5 est une vue en perspective illustrant une autre bague particulière du fixateur de rachis qui est destinée à la fixation de deux ensembles sur le sacrum.
Fig. 6 est une coupe suivant VI-VI (fig. 1) montrant la fixation de la bague représentée en fig. 5 dans le sacrum.

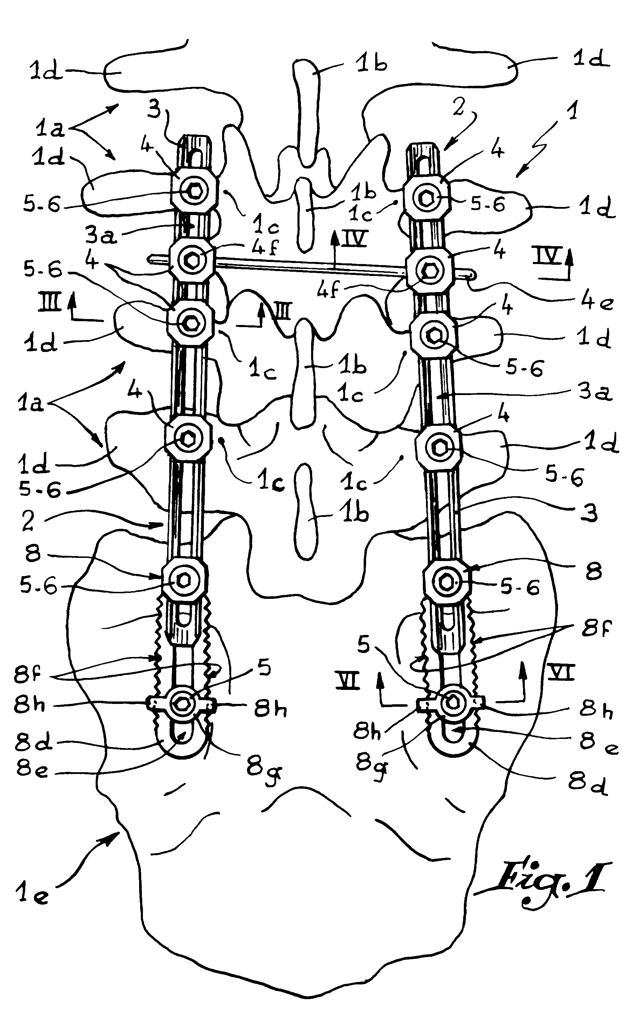

On a représenté en fig. 1 une partie d'une colonne vertébrale 1 comprenant des vertèbres 1a qui sont réunies les unes aux autres par un fixateur 2, afin de réaliser une fusion des étages vertébraux. Le fixateur 2 comprend deux ensembles sensiblement parallèles qui sont placés de part et d'autre des épineuses 1b et immobilisés sur les pédicules 1c des apophyses transverses 1d.

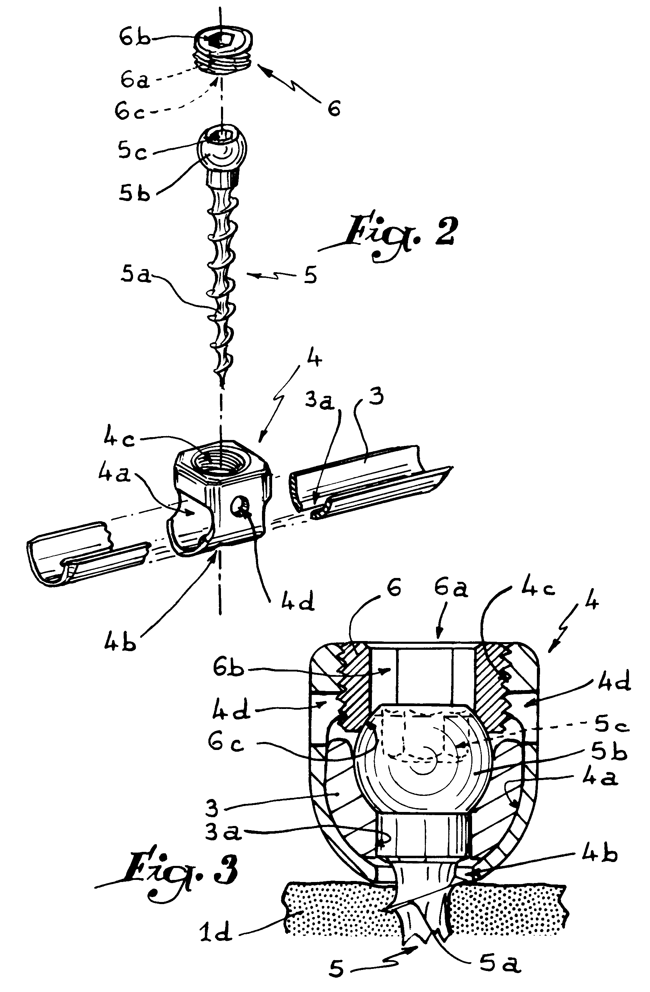

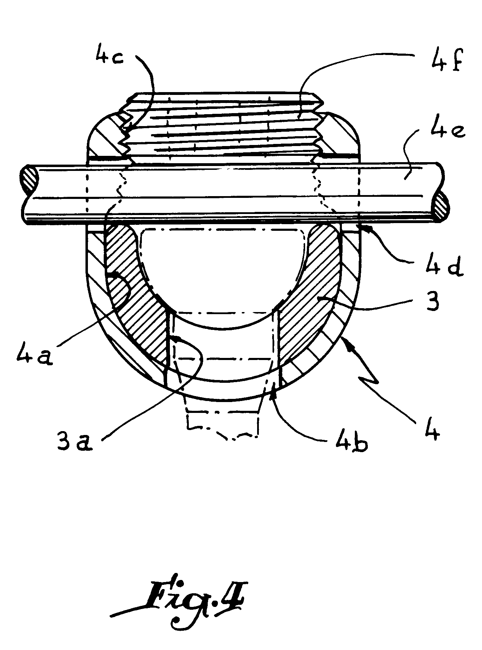

En fig. 2 et 3, on a montré les différents éléments qui composent chaque fixateur 2 et en particulier deux coulisses 3 qui sont généralement cintrées, soit dans le sens convexe, soit dans le sens concave suivant la morphologie du segment rachidien à fixer et la correction à obtenir. Chaque coulisse est choisie d'une longueur adaptée au nombre de vertèbres 1a devant être reliées. Chaque coulisse 3 présente un profil en forme de U dont le fond est percé sur une grande partie de sa longueur d'une lumière débouchante 3a.

Une bague 4 à profil extérieur carré dans sa partie supérieure et arrondi dans sa partie inférieure est percée suivant son axe horizontal d'une ouverture débouchante 4a. Cette dernière présente le même profil interne en forme de U que celui externe de la coulisse 3. Le fond de la bague 4 est percé d'un trou vertical 4b qui débouche à l'extérieur à partir de l'ouverture 4a. Dans la partie supérieure de la bague 4 et à l'opposé du trou 4b et suivant le même axe vertical est prévu un alésage taraudé 4c qui communique avec l'ouverture 4a.

De plus, la bague 4 comprend un trou 4d qui est prévu dans le même plan horizontal que l'ouverture 4a, mais perpendiculairement à celle-ci. Le trou 4d est compris entre l'alésage 4c et l'ouverture 4a.

Une vis 5 permet l'assemblage des éléments constitutifs du fixateur sur chaque vertèbre 1a de la colonne 1. Elle comprend un filet autotaraudant 5a et une tête sphérique 5b qui est percée en son milieu et dans sa partie supérieure d'un trou six pans creux 5c.

Enfin, un capuchon fileté 6 est prévu pour coopérer avec l'alésage taraudé 4c de la bague 4. Ce capuchon est percé d'un trou vertical débouchant 6a qui présente dans sa partie supérieure un profil à six pans creux 6b, tandis que sa partie inférieure forme une portion de sphère 6c de même diamètre que la tête sphérique 5b de la vis 5.

On a représenté en fig. 4 la bague 4 qui est utilisée comme bague complémentaire pour relier entre eux les ensembles du fixateur 2 lors de sa stabilisation transversale.

Le trou 4d est conçu pour recevoir l'une des extrémités d'une tige 4e qui est serrée au moyen de capuchon fileté 4f sur la coulisse 3. L'autre extrémité de la tige 4e est immobilisée de la même manière dans une autre bague 4 qui est prévue sur l'autre ensemble, comme représenté en fig. 1 et 5. Les bagues 4 sont généralement prévues entre deux autres bagues 4 d'un ensemble pour que la tige 4e puisse librement passer entre deux épineuses 1b.

Eventuellement, une vis peut être interposée dans le dispositif de fixation transversale pour améliorer la mise en place du fixateur comme cela est représenté en trait mixte en fig. 4.

La liaison entre les ensembles au moyen de la tige 4e permet de rigidifier le fixateur 2 de rachis.

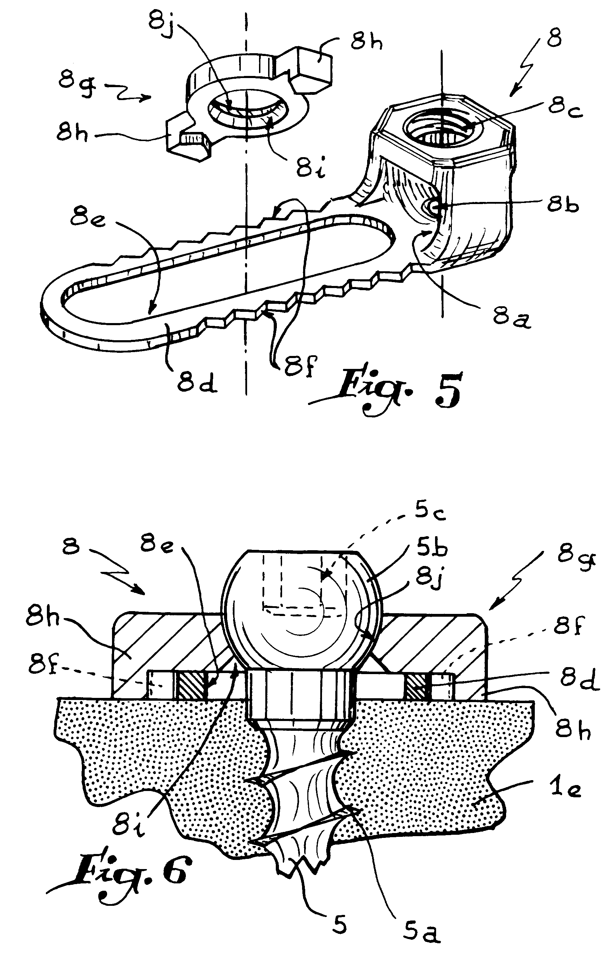

En fig. 5 et 6 on a représenté une bague 8 qui est conçue de la même manière que la bague d'origine 4. La bague 8 comprend une ouverture horizontale 8a en forme de U, un trou débouchant 8b qui est percé dans le fond de ladite bague et un alésage taraudé 8c qui est disposé à l'opposé du trou 8b. L'alésage taraudé 8c reçoit un capuchon fileté identique à celui décrit et référencé 6.

Le fond de la bague 8 est solidaire, dans le prolongement du fond de l'ouverture horizontale 8a, d'une plaque allongée 8d. La plaque 8d est percée d'une lumière oblongue 8e et comprend éventuellement sur chacun de ses bords extérieurs des dents 8f.

Enfin, il est prévu une rondelle 8g qui comprend sur son pourtour deux oreilles opposées 8h pourvues de dents intérieures qui coopèrent lors du montage avec celles de la plaque 8d. La rondelle 8g est percée d'un trou central 8i qui comporte un chanfrein extérieur 8j à profil concave de même rayon que celui de la tête sphérique 5b de la vis 5.

La bague 8 est utilisée dans les cas ou une prise dans le sacrum est nécessaire pour compléter la fixation prévue sur la dernière vertèbre 1a étant donné que la charnière lombo-sacrée est le siège des plus grands efforts.

En effet la bague 8 est fixée sur la première vertèbre du sacrum 1e de la même manière que la bague 4 comme décrit précédemment, tandis que la plaque 8d est immobilisée sur la seconde vertèbre du sacrum 1e par l'intermédiaire de la rondelle 8g et d'une vis pédiculaire 5.

L'opérateur doit procéder de la manière suivante pour la mise en place sur la colonne vertébrale 1 du fixateur 2 décrit précédemment :
- Il prépare le premier ensemble constituant le fixateur 2 en fonction du nombre de vertèbres 1a à immobiliser, c'est-à-dire qu'il introduit sur la coulisse 3 un certain nombre de bagues 4. Certaines bagues 4 sont placées entre deux autres bagues 4 en fonction du nombre de raccordements transversal que l'opérateur désire réaliser entre les deux ensembles.
- Il introduit à l'intérieur de chaque bague 4 une vis 5 qui vient coopérer avec la lumière 3a de la coulisse 3 et le trou 4b.
- Il serre les vis 5 à l'intérieur des pédicules 1c de chaque vertèbre 1a.
- Il visse les capuchons filetés 6 dans les alésages taraudés 4c des bagues 4 de manière qu'ils viennent prendre appui sur la partie supérieure de la tête 5b des vis 5. Simultanément, le serrage des capuchons 6 permet de venir bloquer la coulisse 3 et les bagues 4 contre la partie inférieure de la tête cylindrique 5b des vis 5 (fig. 3).
- Il procède de la même façon pour la mise en place du second ensemble du fixateur 2.
- Il visse ensuite les capuchons filetés 4f des bagues complémentaires 4 des deux ensembles pour immobiliser les tiges 4e préalablement introduites dans les trous 4d afin de rigidifier le fixateur 2 (fig. 1 et 3).

Lorsque pour des problèmes de pathologie, l'opérateur doit assembler le fixateur sur le sacrum 1e, il utilise une bague 8 décrite précédemment.

On constate que la structure particulière des bagues 8 permet de réduire la hauteur d'encombrement à l'endroit où la colonne vertébrale n'est recouverte que par une fine épaisseur de peau.

On remarque que les différentes bagues 4 et 8 enserrent les vis pédiculaires 5 et la coulisse 3 en donnant une rigidité à l'assemblage puisqu'elles limitent la déformation de la coulisse 3 pendant le serrage des vis 5.

La solidité de la jonction entre vis et coulisse est assurée de façon directe par le serrage des capuchons sur la tête des vis et de façon indirecte par la déformation élastique des bagues reserrant latéralement la coulisse sur l'embase sphérique des vis.

On note que les ensembles composant le fixateur de rachis peuvent être utilisés uniquement pour la solidarisation de vertèbres 1a entre elles sans l'emploi de la bague 8.

Le fixateur du rachis suivant la présente invention permet d'obtenir une souplesse relative entre les vertèbres instrumentées complétées à une fixation très stable dans les vertèbres.

## Revendications

1. Fixateur de rachis constitué de deux ensembles sensiblement parallèles pour l'alignement et le maintien d'une colonne vertébrale, comprenant chacun une coulisse, des vis pédiculaires à filet auto-taraudant à tête sphérique et des capuchons filetés, caractérisé en ce qui chacun des ensembles est constitués :
- d'une coulisse (3) qui présente une forme en U dont le fond est percé d'une lumière débouchante (3a) ;
- de plusieurs bagues (4) qui sont chacune percées d'une ouverture horizontale débouchante (4a) présentant un profil interne correspondant à celui de la coulisse, d'un alésage taraudé (4c) coaxial à un trou lisse (4b) ménagé dans le fond de ladite bague perpendiculairement à l'ouverture (4a) ;
- de vis pédiculaires (5) à filet auto-taraudant (5a) et à tête sphérique (5b) ;
- et de capuchons filetés (6) vissés dans l' alésage taraudé (4c) de chaque bague (4) et qui prennent appui chacun sur la partie supérieure de la tête sphérique (5b) de la vis pédiculaire (5) de manière à assembler par serrage la bague (4) et la coulisse (3) par l'intermédiaire de la tête sphérique (5b) de ladite vis (5).

2. Fixateur suivant la revendication 1, caractérisé en ce que la bague (4) est pourvue d'un trou débouchant (4d) qui est prévu au-dessus de l'ouverture (4a), mais perpendiculairement à celle-ci.

3. Fixateur suivant la revendication 2, caractérisé en ce que le trou (4d) d'une première bague (4) complémentaire est conçu pour recevoir l'une des extrémités libres d'une tige (4e) qui est immobilisée par serrage entre un capuchon taraudé (4f) et la coulisse (3), tandis que l'autre extrémité de la tige (4e) est fixée de la même manière, mais dans une seconde bague (4) complémentaire.

4. Fixateur suivant la revendication 1, caractérisé en ce qu'il comprend une bague (8) pourvue d'une ouverture horizontale débouchante (8a) en forme de U, un trou (8b) percé dans le fond de ladite bague, un alésage taraudé (8c) coaxial audit trou (8b) et une plaque hozirontale (8d) qui est prévue au niveau du fond de ladite bague (8) et dans le prolongement de l'ouverture (8a).

5. Fixateur suivant la revendication 4, caractérisé en que la plaque (8d) est percée d'une lumière oblongue (8e) et comporte sur sa périphérie extérieure des dents (8f) qui coopérent au montage avec deux oreilles opposées (8h) solidaires d'une rondelle (8g).

6. Fixateur suivant la revendication 1, caractérisé en ce que le capuchon taraudé (6) comporte dans sa partie inférieure une portée (6c) en portion de sphère de même rayon que la tête sphérique (5b) de la vis pédiculaire (5).

7. Fixateur suivant la revendication 5, caractérisé en que la rondelle (8g) est percée d'un trou central (8i) qui comporte un chanfrein extérieur (8j) à profil concave de même rayon que celui de la tête sphérique (5b) de la vis (5).

8. Fixateur suivant les revendications 1, 2 et 4, caractérisé en ce que les bagues (4, 8) présentent un profil extérieur carré dans leur partie supérieure et arrondi dans leur partie inférieure.

## Claims

1. Spinal fixator consisting of two substantially parallel assemblies for aligning and holding a vertebral column, each comprising a slide rail, pedicle screws with self-tapping thread and spherical head, and threaded caps, characterized in that each of the assemblies consists of:
- a slide rail (3) which has a U shape, the bottom of which is punched with an open slot (3a);
- several rings (4) which are each punched with a horizontal through-opening (4a) having an internal profile corresponding to that of the slide rail, a tapped bore (4c) coaxial to a smooth hole (4b) formed in the bottom of the said ring perpendicular to the opening (4a);
- pedicle screws (5) with self-tapping thread (5a) and spherical head (5b);
- and threaded caps (6) screwed into the tapped bore (4c) of each ring (4) and which each bear on the upper part of the spherical head (5b) of the pedicle screw (5) in such a way as to assemble, by clamping, the ring (4) and the slide rail (3) by way of the spherical head (5b) of the said screw (5).

2. Fixator according to Claim 1, characterized in that the ring (4) is equipped with an open hole (4d) which is provided above the opening (4a), but perpendicular thereto.

3. Fixator according to Claim 2, characterized in that the hole (4d) of a first complementary ring (4) is designed to receive one of the free ends of a rod (4e) which is immobilized, by clamping, between a tapped cap (4f) and the slide rail (3), while the other end of the rod (4e) is fixed in the same way, but in a second complementary ring (4).

4. Fixator according to Claim 1, characterized in that it comprises a ring (8) equipped with a horizontal through-opening (8a) of U shape, a hole (8b) punched in the bottom of the said ring, a tapped bore (8c) coaxial to the said hole (8b), and a horizontal plate (8d) which is provided level with the bottom of the said ring (8) and in the continuation of the opening (8a).

5. Fixator according to Claim 4, characterized in that the plate (8d) is punched with an oblong slot (8e) and includes, on its outer periphery, teeth (8f) which cooperate upon assembly with two opposite lugs (8h) integral with a washer (8g).

6. Fixator according to Claim 1, characterized in that the tapped cap (6) includes, in its lower part, a bearing surface (6c) in the form of a sphere portion with the same radius as the spherical head (5b) of the pedicle screw (5).

7. Fixator according to Claim 5, characterized in that the washer (8g) is punched with a central hole (8i) which includes an outer chamfer (8j) of concave profile with the same radius as that of the spherical head (5b) of the screw (5).

8. Fixator according to Claims 1, 2 and 4, characterized in that the rings (4, 8) have an outer profile which is square in their upper part and rounded in their lower part.

## Patentansprüche

1. Rückgratstütze zum Ausrichten und Aufrechthalten einer Wirbelsäule, die aus zwei nahezu parallelen Einheiten aufgebaut ist, die jeweils eine Profilschiene, pedikuläre Knochenschrauben mit einem selbst-schneidenden Gewinde und einem kugelförmigen Kopf, sowie Gewindeeinsätze aufweisen
**dadurch gekennzeichnet**, **daß**
jede Einheit besteht aus:
- einer U-förmigen Profilschiene (3), deren Bodenfläche von einer aufnehmenden Nut (3a) durchsetzt ist;
- mehreren Hülsen (4), die jeweils von einer horizontalen Aufnahmeöffnung (4a) mit einem der Profilschiene entsprechendem Innenprofil und von einer zu einer glatten Bohrung (4b), die sich in der Bodenfläche dieser Hülse und senkrecht zur Aufnahmeöffnung (4a) befindet, koaxialen Gewindebohrung (4c) durchsetzt sind;
- pedikulären Knochenschrauben (5) mit einem selbst-schneidenden Gewinde (5a) und einem kugelförmigen Kopf (5b);
- und Gewindeeinsätzen (6), die in die Gewindebohrung (4c) einer jeden Hülse (4) geschraubt sind und die jeweils auf dem oberen Abschnitt des kugelförmigen Kopfes (5b) der pedikulären Knochenschrauben (5) so zur Auflage kommen, daß die Hülse (4) und die Profilschiene (3) mit Hilfe des kugelförmigen Kopfes (5b) dieser pedikulären Knochenschraube (5) miteinander verspannt sind.

2. Stütze nach Anspruch 1,
**gekennzeichnet durch**
eine Hülse (4), die mit einem Aufnahmeloch (4d) versehen ist, das oberhalb, aber senkrecht zur Aufnahmeöffnung (4a) vorgesehen ist.

3. Stütze nach Anspruch 2,
**gekennzeichnet durch**
ein Aufnahmeloch (4d) einer ersten komplementären Hülse (4), das dazu dient, eines der freien Enden eines Gestänges (4e) aufzunehmen, das über das Verspannen zwischen dem Gewindeeinsatz (4f) und der Profilschiene (3) unbeweglich gehalten ist, währenddessen das andere Ende des Gestänges (4e) auf diesselbe Weise, aber in einer zweiten komplementären Hülse (4) befestigt ist.

4. Stütze nach Anspruch 1,
**gekennzeichnet durch**
eine Hülse (8), die mit einer horizontalen U-förmigen Aufnahmeöffnung (8a), einem die Bodenfläche dieser Hülse durchdringenden Loch (8b), einer zu diesem Loch (8b) koaxial ausgerichteten Gewindebohrung (8c) und einer horizontalen Zunge (8d) versehen ist, die auf Bodenhöhe dieser Hülse (8) und in Verlängerung zur Aufnahmeöffnung (8a) angebracht ist.

5. Stütze nach Anspruch 4,
**gekennzeichnet durch**
eine Zunge (8d), die von einem Langloch (8e) durchsetzt ist und an ihrem Außenrand Zähne (8f) aufweist, die im montierten Zustand mit zwei gegenüberliegenden und mit einem Feststellring (8g) fest verbundenen Nasen (8h) zusammenwirken.

6. Stütze nach Anspruch 1,
**gekennzeichnet durch**
einen Gewindeeinsatz (6), der im unteren Abschnitt eine Fase (6c) in Form einer Kugelfläche mit demselben Radius des kugelförmigen Kopfes (5b) der pedikulären Knochenschraube (5) aufweist.

7. Stütze nach Anspruch 5
**gekennzeichnet durch**
einen Feststellring (8g), der von einer Zentrierbohrung (8i) durchsetzt ist, die eine Außenfase (8j) in einem konkaven Profil mit demselben Radius des kugelförmigen Kopfes (5b) der pedikulären Knochenschraube (5) aufweist.

8. Stütze nach den Ansprüchen 1, 2 und 4,
**gekennzeichnet durch**
Hülsen (4, 8), die im oberen Abschnitt ein eckiges und im unteren Abschnitt ein abgerundetes Außenprofil aufweisen.
